# EUROPEAN PATENT APPLICATION

(11) **EP 3 939 612 A1**
(43) Date of publication of application: **19.01.2022**
(21) Application number: 20769042.1
(22) Date of filing: 03.03.2020
(51) Int. Cl.: A61K 45/00, A61P 35/00, A61P 43/00, A61K 47/52, C01B 32/15, C01B 32/28, A61K 33/44, A61K 41/00

(54) **OPTICALLY HEAT-GENERATING COMPOSITE MATERIAL, NANOCLUSTER, SUBSTANCE DELIVERY CARRIER AND PHARMACEUTICAL COMPOSITION**

(30) Priority: 13.03.2019 JP 2019045505
(71) Applicant: NATIONAL INSTITUTE OF ADVANCED INDUSTRIAL SCIENCE AND TECHNOLOGY, Chiyoda-ku Tokyo 100-8921 (JP); Daicel Corporation, Osaka 530-0011 (JP)
(72) Inventor: MIYAKO, Eijiro, Tsukuba-shi, Ibaraki 305-8560 (JP); YU, Yue, Tsukuba-shi, Ibaraki 305-8560 (JP); NISHIKAWA, Masahiro, Tokyo 108-8230 (JP); LIU, Ming, Tokyo 108-8230 (JP); TEI, Takahiro, Tokyo 108-8230 (JP)
(74) Representative: Grünecker Patent- und Rechtsanwälte PartG mbB
(86) International application number: PCT/JP2020/008812
(87) International publication number: WO 2020/184271

(57) **Abstract**

The present invention provides a photo-exothermic composite material represented by Formula (I) of CNM-(Y¹-R)ₙ₁ (I) (wherein, CNM denotes a carbon nanomaterial, Y¹ denotes a divalent linking group, R denotes a group derived from a fluorescent substance or pigment; and n1 is an integer of 1 or greater).

## Description

### Technical Field

The present invention relates to a photo-exothermic composite material, a nanocluster, a substance delivery carrier and a pharmaceutical composition.

The following abbreviations are used in the present specification and drawings.
ND: nanodiamond
CND: carbon nanodiamond
CNDp: carbon nanodiamond particle
SP: super nanoparticle (or cluster)
ICG: Indocyanine Green
PTX: Paclitaxel
MES: 2-(N-morpholino)ethanesulfonate
ND-ori: A raw material (original nanodiamond)
PTX@ICG-ND-SP: A complex of PTX and a cluster (SP) configured from ICG-modified ND
ICG-ND-SP: Nanoclusters in which a CND composite material to which ICG is bonded is self-assembled

In the present specification, ND may be used synonymously with CND

### Background Art

In addition to the inherent properties of diamonds, nanodiamonds (referred to hereinafter as "ND") also exhibit characteristics including a small average particle diameter and a large specific surface area, and provide merits such as being relatively inexpensive and easy to obtain. ND can be manufactured through an explosion method or a high-temperature / high-pressure method. ND also exhibit low toxicity, superior biocompatibility and stable fluorescence properties, and therefore applications in the biomedical field are being widely studied.

In addition to the inherent properties of diamonds, carbon nanodiamonds (referred to hereinafter as "CND") also exhibit characteristics including a small average particle diameter and a large specific surface area, and provide merits such as being relatively inexpensive and easy to obtain. CND can be manufactured by methods such as an explosion method or a high-temperature/high-pressure method (Patent Document 1). CND also exhibit low toxicity, superior biocompatibility, and stable fluorescence properties, and therefore applications in the biomedical field are being widely studied. Furthermore, Patent Documents 2 and 3 disclose modified CND and methods for manufacturing the same.

Although research pertaining to cancer hypothermia treatments using the photo-exothermic effect of CND has already been reported (Non-Patent Document 1), the photo-exothermic effect of the CND itself is very weak.

### Citation List

### Patent Document

Patent Document 1: JP 2010-202458 A
Patent Document 2: JP 2017-186234 A
Patent Document 3: JP 2017-186235 A

### Non-Patent Literature

Non-Patent Document 1: T. -K. Ryu, S. W. Baek, R. H. Kang, S. -W. Choi, Adv. Funct. Mater. 2016, 26, 6428.

### Summary of Invention

### Technical Problem

A main object of the present invention is to provide a material that has a strong photo-exothermic effect and can be used in applications such as the treatment of tumors.

### Solution to Problem

The present invention provides the following photo-exothermic composite material, nanocluster, substance delivery carrier and pharmaceutical composition.
1. A photo-exothermic composite material represented by Formula (I) below: CNM-(Y¹-R)ₙ₁ (I)
   (wherein, CNM denotes a carbon nanomaterial; Y¹ denotes a divalent linking group; R denotes a group derived from a fluorescent substance or pigment; and n1 is an integer of 1 or greater).
2. The photo-exothermic composite material according to 1, wherein the divalent linking group denoted by Y¹ is selected from the group consisting of -NH-, - O-, -CO-O-, -O-CO-, -CO-NH-, -NH-CO-, -NH-CO-O-, -O-CO-NH-,-O-CO-O-, and -NH-CO-NH-.
3. The photo-exothermic composite material according to 1 or 2, wherein the carbon nanomaterial is a carbon nanodiamond or carbon nanodots.
4. The photo-exothermic composite material according to 3, wherein the carbon nanomaterial is a carbon nanodiamond.
5. The photo-exothermic composite material according to any one of 1 to 4,
   wherein the carbon nanomaterial has at least one surface group selected from the group consisting of OH, COOH, and NH₂, and the group derived from a fluorescent substance or pigment and denoted by R is bonded to the carbon nanomaterial through the surface group.
6. A nanocluster in which the photo-exothermic composite material described in any of 1 to 5 is self-assembled.
7. The nanocluster according to 6, further complexed with an active ingredient.
8. The nanocluster according to 7, wherein the active ingredient is a physiologically active substance, a fluorescent substance, or a pigment.
9. A delivery carrier for an active ingredient, the delivery carrier containing the nanocluster described in 7 or 8.
10. A pharmaceutical composition containing the nanocluster described in 7 or 8, the nanocluster being complexed with a drug.

### Advantageous Effects of Invention

The composite material of the present invention exhibits an excellent photo-exothermic property, and therefore when the composite material is distributed to a tumor site and irradiated with light, the composite material can exhibit an anti-tumor effect. Also, nanoclusters further containing an anti-tumor agent are useful as pharmaceutical compositions. The photo-exothermic property can be further enhanced by including a fluorescent substance and pigment in the nanoclusters.

### Brief Description of Drawings

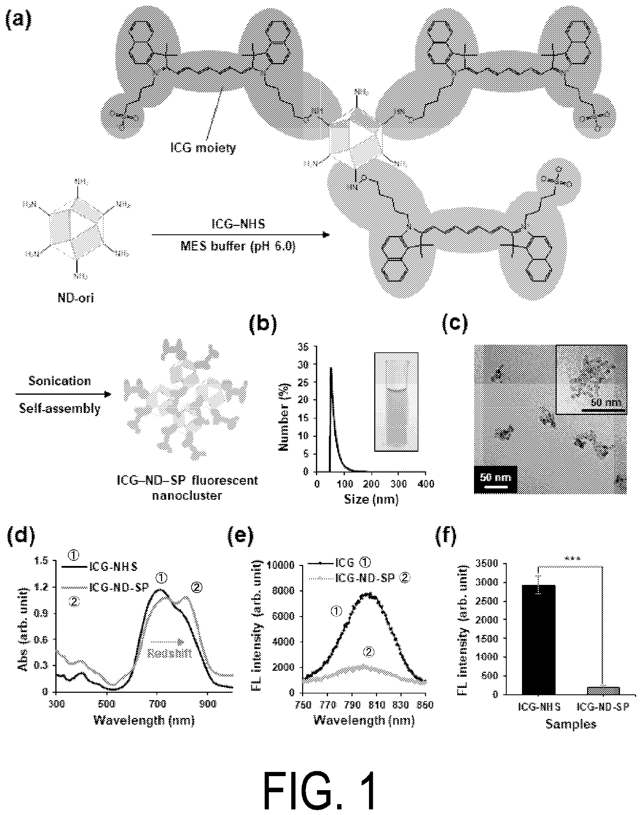

FIG. 1 illustrates the synthesis and characterization of ICG-ND-SP. FIG. 1(a) outlines a scheme for synthesizing ICG-ND-SP. An amino group of ND-ori and a succinimidyl group of ICG-NHS are covalently bonded through a dehydration condensation reaction, after which a self-assembled ICG-ND-P nanocluster (ICG-ND-SP fluorescent nanocluster) is prepared by sonication. FIG. 1(b) is a graph showing a size distribution analysis of the ICG-ND-SP through dynamic light scattering (DLS). FIG. 1(c) is a transmission electron microscope (TEM) image of the ICG-ND-SP. The top right photograph is a highly magnified TEM image of the ICG-ND-SP. FIG. 1(d) is a graph showing a UV-Vis-NIR absorption spectral analysis of an ICG-ND-SP dispersed aqueous solution (ICG concentration = 18.75 µg ml⁻¹, ND concentration = 75 µg ml⁻¹) and of ICG-NHS (ICG concentration = 18.75 µg ml⁻¹). FIG. 1(e) is a graph showing the fluorescence (FL) intensity of the ICG-ND-SP dispersed aqueous solution and an ICG-NHS (ICG concentration = 2 µg ml⁻¹) aqueous solution. FIG. 1(f) is a graph showing the FL intensity analysis of an ICG-NHS aqueous solution and an ICG-ND-SP dispersed aqueous solution in which the ICG concentration was adjusted in advance to 0.75 µg ml⁻¹. The ND concentration was 3 µg ml⁻¹.

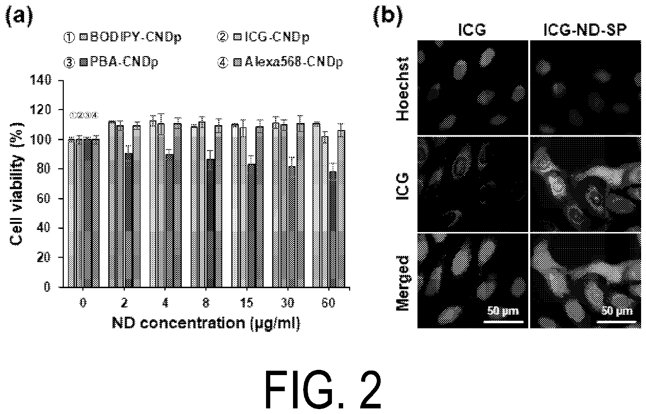

FIG. 2 illustrates the intracellular penetration and distribution behavior of ICG-ND-SP. FIG. 2(a) is a graph showing the cytotoxicity of a variety of fluorescent molecule-modified ND-SP. Cytotoxicity was evaluated after incubation of various ND-SP in human osteosarcoma cells (U2OS) for 24 hours. FIG. 2(b) presents fluorescence microscope images of U2OS cells when ICG (ICG concentration = 1.5 µg ml⁻¹) or ICG-ND-SP (ICG and ND concentrations adjusted to 1.5 and 6 µg ml⁻¹, respectively) was incubated for 4 hours in a 37°C, 5% CO₂ environment. The cells were washed with PBS and then fixed with 4% paraformaldehyde (PFA).

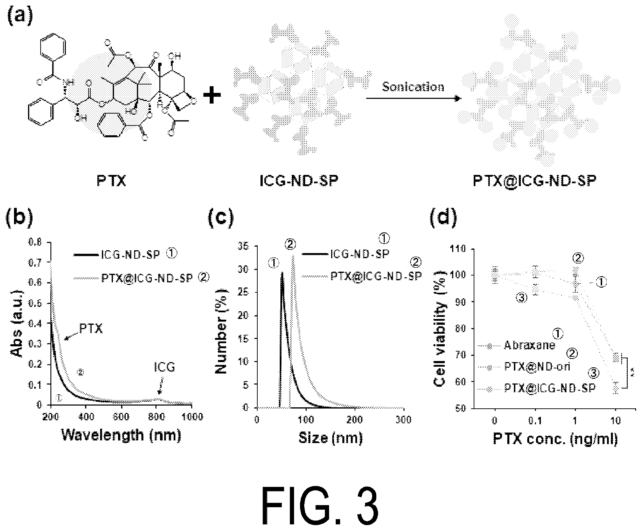

FIG. 3 illustrates the in vitro anticancer effect of PTX@ICG-ND-SP as a drug carrier. FIG. 3(a) outlines a method for synthesizing PTX@ICG-ND-SP using noncovalent interaction between PTX and ICG-ND-SP. FIG. 3(b) is a graph showing the UV-Vis-NIR absorption spectra of ICG-ND-SP before being complexed with PTC (ICG-ND-SP) and after being complexed with PTX (PTX@ICG-ND-SP). FIG. 3(c) is a graph showing DLS measurement results of ICG-ND-SP and PTX@ICG-ND-SP. FIG. 3(d) is a graph showing the anticancer activity of Abraxane (an FDA approved and commercially available PTX-based nanomedicine), PTX@ND-ori, and PTX@ICG-ND-SP against human ovarian cancer cells (SKOV3) after 24 hours of incubation.

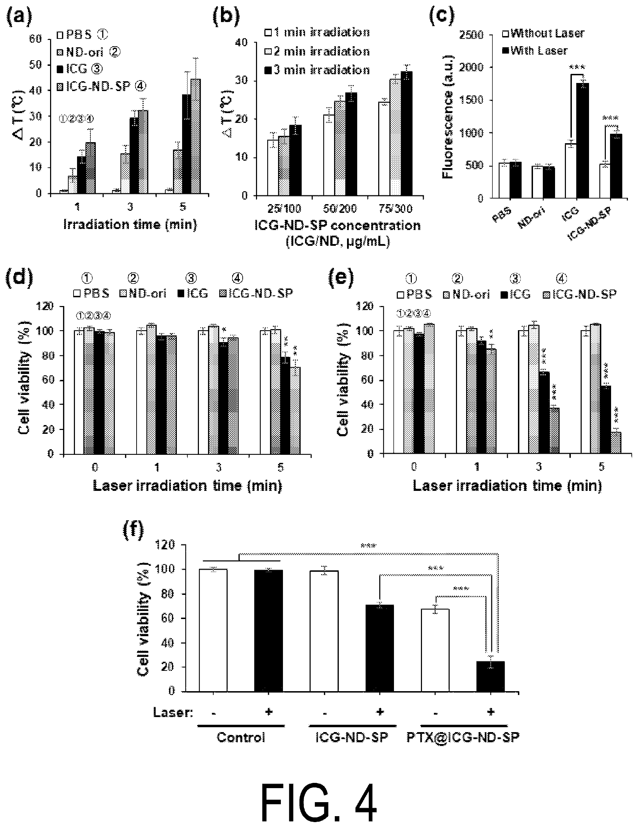

FIG. 4 presents graphs of evaluations of the in vitro anticancer activity of photo-introduced ICG-ND-SP. FIG. 4(a) is a graph of temperature measurements of a PBS buffer solution, ND-ori, ICG, and ICG-ND-SP, which were irradiated with a laser, the temperature being measured at each laser irradiation time. The ICG and ND concentrations were set to 0.75 and 3 mg ml⁻¹, respectively. FIG. 4(b) is a graph showing the impact of the ICG-ND-SP concentration on the increase in temperature at each laser irradiation time. FIG. 4(c) is a graph showing the ROS generation behavior from the PBS buffer solution, ND-ori, ICG, and ICG-ND-SP when irradiated and not irradiated with a laser. The ICG and ND concentrations were set to 75 and 300 µg ml⁻¹, respectively. FIG. 4(d) is a graph showing the cell viability percentage of SKOV3 cells at each laser irradiation time of the PBS buffer solution, ND-ori, ICG, and ICG-ND-SP, which were irradiated with a laser. FIG. 4(e) is a graph showing the cell viability percentage after 24 hours following laser irradiation. The ICG and ND concentrations were set to 25 and 100 µg ml⁻¹, respectively. FIG. 4(f) is a graph showing the viability percentage of SKOV3 cells exposed to ICG-ND-SP and PTX@ICG-ND-SP incubated for 24 hours after laser irradiation for 3 minutes. The PTX, ICG, and ND concentrations were set to 10, 75, and 300 µg ml⁻¹, respectively.

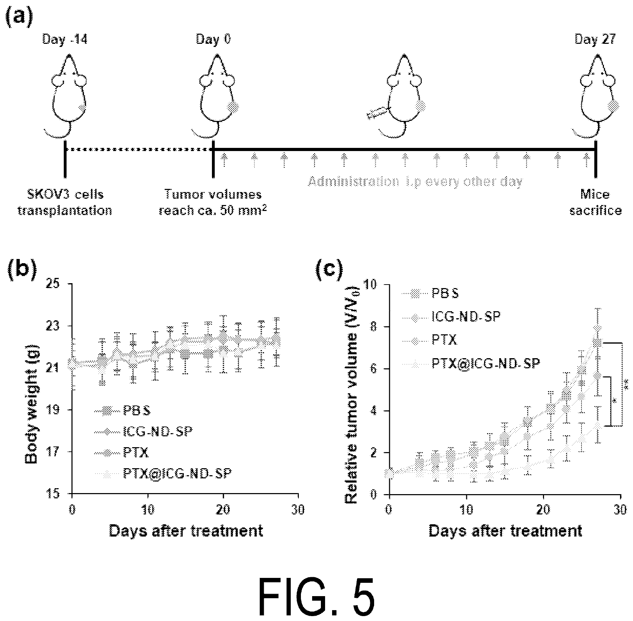

FIG. 5 illustrates the in vivo anticancer action of ICG-ND-SP as a drug carrier. FIG. 5(a) outlines a method for an in vivo anticancer experiment using ICG-ND-SP. FIG. 5(b) is a graph of the body weight of mice subjected to different treatments, and FIG. 5(c) is a graph of the solid cancer volume with the different treatments. The PBS buffer solution and sterile water in which was dispersed ND-ori, ICG-ND-SP, or PTX@ICG-ND-SP was administered into the abdominal cavity. The solutions were administered with the dosage concentrations set to 5 mg kg⁻¹ for the PTX, 7.5 mg kg⁻¹ for the ICG, and 30 mg kg⁻¹ for the ND

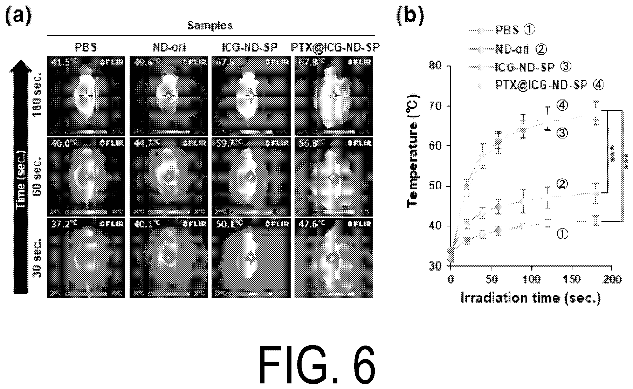

FIG. 6(a) is thermal images of the mouse body surface when irradiated with a 785 nm laser (laser output = 1 W (up to 80 mW/mm²), laser irradiation time = 180 sec) and administered PBS, ND-ori, ICG-ND-SP or PTX@ICG-ND-SP. FIG. 6(b) is a graph of the temperature of the solid cancer. The dosage concentrations were set to 1.25 mg kg⁻¹ for the PTX, 1.88 mg kg⁻¹ for the ICG, and 7.5 mg kg⁻¹ for the ND. The mouse body surface at the time of thermal measurements was approximately 33°C.

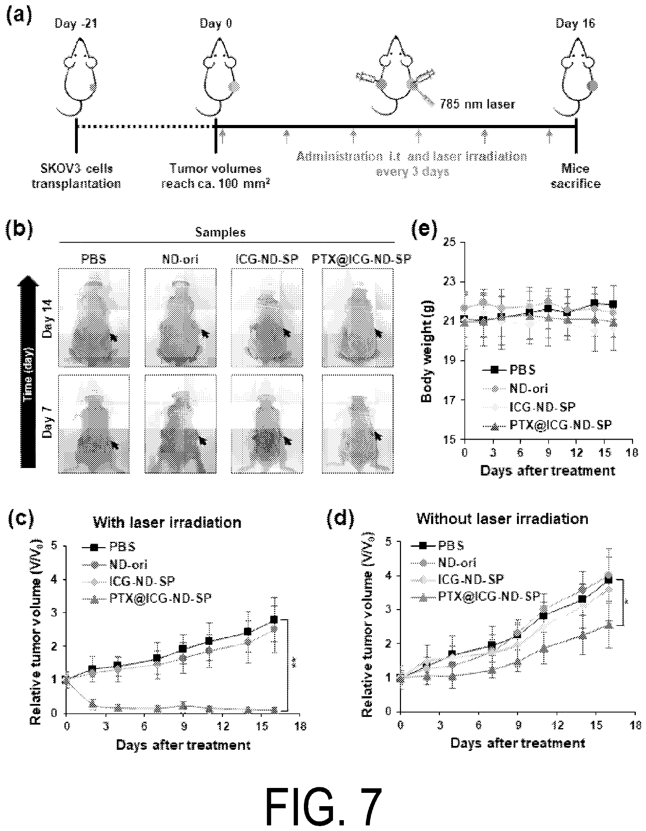

FIG. 7 illustrates an in vivo verification of the photo-exothermic action, photodynamic action, and pharmacotherapeutic action. FIG. 7(a) outlines an experimental design for verifying multidimensional anticancer effects using ICG-ND-SP. FIG. 7(b) presents photographs after 7 days and after 14 days of mice in which PBS, ND-ori, ICG-ND-SP, or PTX@ICG-ND-SP was administered directly to the solid cancer (SKOV3), and the solid cancer on the right side was irradiated with a laser (laser intensity = 1 W (up to 80 mW/mm²), laser irradiation time = 180 sec). FIG. 7(c) is a graph showing the size of the solid cancer on the right side, which was irradiated with a 785 nm laser. FIG. 7(d) is a graph of the size of the solid cancer on the left side, which was not irradiated with a laser. FIG. 7(e) is a graph of the transition in body weight after laser irradiation. The dosage concentrations were set to 1.25 mg kg⁻¹ for the PTX, 1.88 mg kg⁻¹ for the ICG, and 7.5 mg kg⁻¹ for the ND

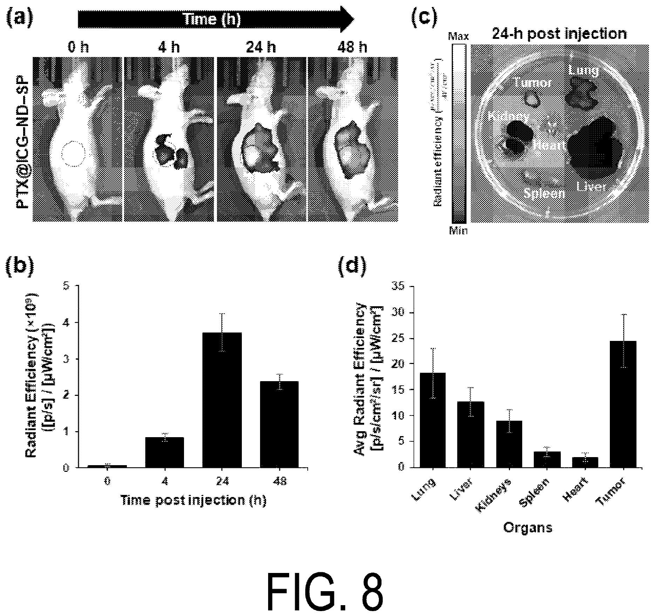

FIG. 8 illustrates the in vivo distribution behavior of PTX@ICG-ND-SP obtained through near-infrared fluorescence bio-imaging. FIG. 8(a) presents fluorescence images of a mouse having solid cancer of SKOV3 cells and for which PTX@ICG-ND-SP (PTX concentration = 5 mg kg⁻¹, ICG concentration = 7.5 mg kg⁻¹, ND concentration = 30 mg kg⁻¹) was administered through the caudal vein. The dashed circle indicates the position of the solid cancer. FIG. 8(b) is a graph of the radiation efficiency corresponding to the solid cancer at different times along the time axis. FIG. 8(c) is an ex vivo fluorescence image of isolated solid cancer and organs. FIG. 8(d) is a graph showing the quantitative radiation efficiency with respect to the solid cancer and organs at 24 hours after the administration of PTX@ICG-ND-SP.

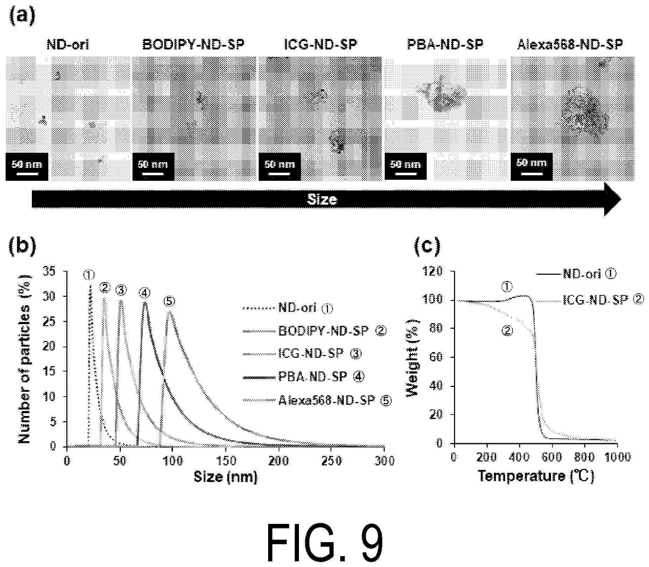

FIG. 9 illustrates the synthesis and characterization of ND-SP with various chemically modified fluorescent pigments. FIG. 9(a) is TEM images of ND-ori and ND-SP with chemically modified fluorescent pigments. FIG. 9(b) is a graph showing the DLS analysis of ND-ori and ND-SP with chemically modified fluorescent pigments. FIG. 9(c) is a graph of TGA curves for ND-ori and ICG-ND-SP.

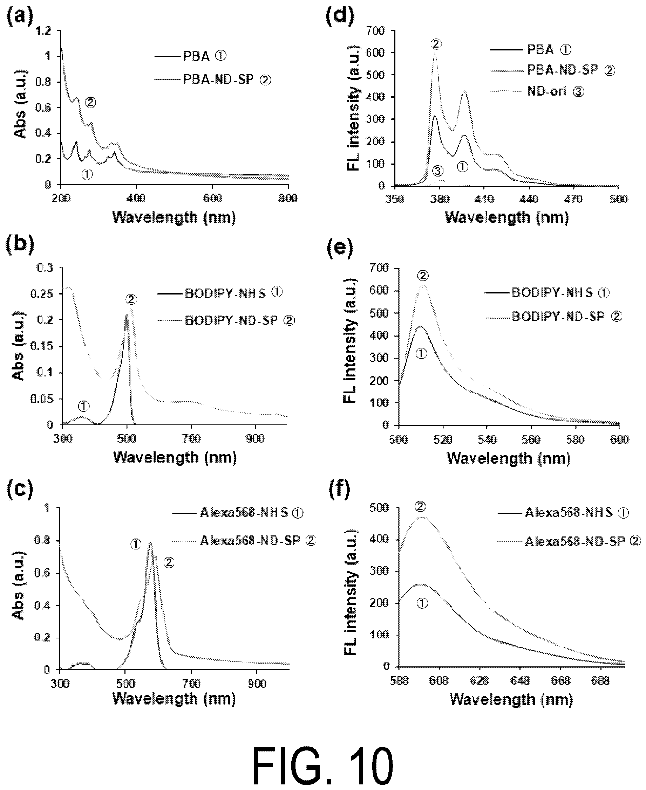

FIG. 10 presents graphs of evaluations of the optical characteristics of ND-SP with various chemically modified fluorescent pigments. FIGS. 10(a) to (c) are graphs of UV-Vis-NIR absorption spectra of various ND-SP. In FIG. 10(a), the PBA (1-pyrenebutyric acid) concentration was 14.4 µg ml⁻¹. The concentrations of PBA and ND in the PBA-ND-SP were set to 1.5 and 30 µg ml⁻¹, respectively. In FIG. 10(b), the BODIPY-NHS concentration was 1.34 µg ml⁻¹. The concentrations of BODIPY and ND in BODIPY-ND-SP were set to 1 and 75 µg ml⁻¹, respectively. In FIG. 10(c), the concentration of Alexa568-NHS was 6.9 µg ml⁻¹. The concentrations of Alexa568 and ND in the Alexa568-ND-SP were set to 4 and 30 µg ml⁻¹, respectively. FIGS. 10(d) to (f) are graphs of the fluorescence (FL) intensity of various ND-SP. In FIG. 10(d), the PBA concentration was 0.14 ng ml⁻¹. The concentrations of PBA and ND in the PBA-ND-SP were set to 3 and 60 ng ml⁻¹, respectively. In FIG. 10(e), the BODIPY-NHS concentration was 5 ng ml⁻¹. The concentrations of BODIPY and ND in the BODIPY-ND-SP were set to 40 and 3000 ng ml⁻¹, respectively. In FIG. 10(f), the Alexa568-NHS concentration was 5 ng ml⁻¹. The concentrations of Alexa568 and ND in the Alexa568-ND-SP were set to 20 and 1500 ng ml⁻¹, respectively.

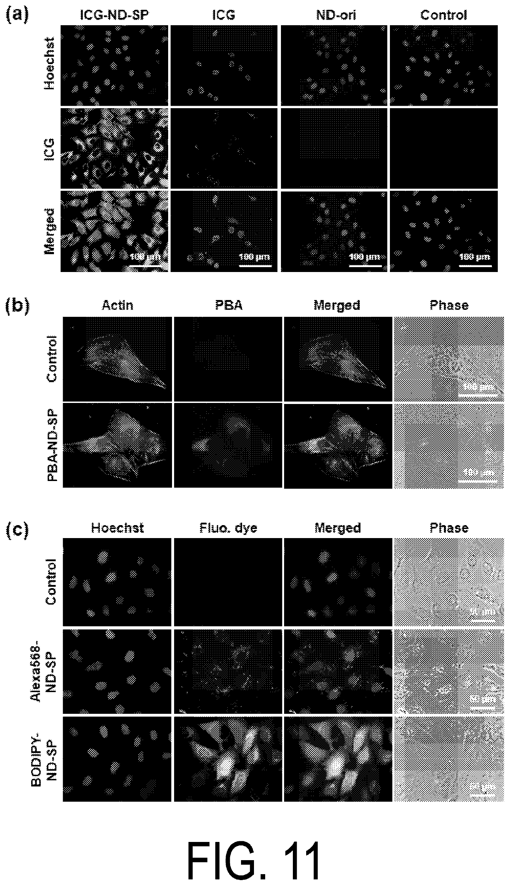

FIG. 11 presents images of fluorescence microscope observations of various ND-SP. FIG. 11(a) presents near infrared fluorescent live imaging of ICG-ND-SP (ICG concentration = 1.5 µg ml⁻¹, ND concentration = 6 µg ml⁻¹), ICG (1.5 µg ml⁻¹), and ND-ori (ND concentration = 6 µg ml⁻¹) after incubation with U2OS cells in a 5% CO₂ atmosphere for 4 hours at 37°C. A control experiment in which a fluorescent pigment was not used was also conducted under the same conditions. FIG. 11(b) shows fluorescence microscope images of U2OS cells when PBA-ND-SP (PBA concentration = 0.3 µg ml⁻¹, ND concentration = 6 µg ml⁻¹) was incubated for 24 hours. FIG. 11(c) presents fluorescence microscope images of U2OS cells when Alexa568-ND-SP (Alexa568 concentration = 0.8 µg ml⁻¹, ND concentration = 60 µg ml⁻¹) or BODIPY-ND-SP (BODIPY concentration = 0.8 µg ml⁻¹, ND concentration = 60 µg ml⁻¹) was incubated for 6 hours. The nuclei and cytoskeletons (actin) were stained using Hoechst 33342 and Phallodin, respectively. The cells shown in FIGS. 11(b) and (c) were fixed with 4% PFA.

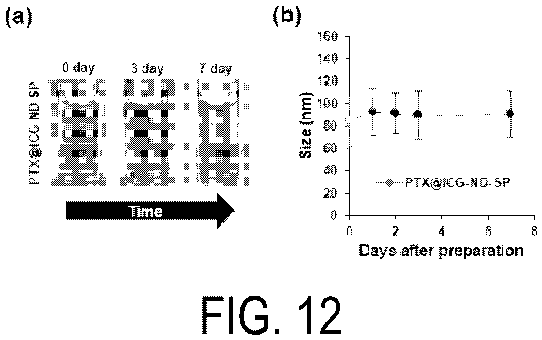

FIG. 12 illustrates the dispersion stability of PTX@ICG-ND-SP in water. FIG. 12(a) is photographs when PTX@ICG-ND-SP (PTX, ICG, and ND-ori concentrations of 0.2, 0.06, and 0.6 mg ml⁻¹, respectively) was incubated for 7 days in MilliQ water. FIG. 12(b) is a graph of DLS analysis results at various points in time (0, 1, 2, 3 and 7 days) of the PTX@ICG-ND-SP aqueous solution.

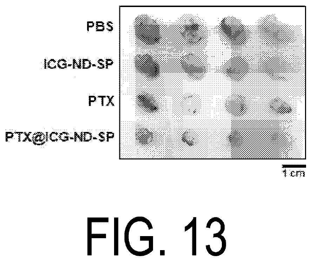

FIG. 13 is a photograph of solid cancers isolated after the end of the experiment.

### Description of Embodiments

In the present specification, the carbon nanomaterials (CNM) used as raw materials for the production of photo-exothermic composite materials include carbon nanodiamonds and carbon nanodots.

A photo-exothermic composite material according to an embodiment of the present invention is represented by Formula (I) below:

CNM-(Y¹R)ₙ₁ (I)

(wherein, CNM denotes a carbon nanomaterial; Y¹ denotes a divalent linking group; R denotes a group derived from a fluorescent substance or pigment; and n1 is an integer of 1 or greater).

Here, n1 denotes an integer of 1 or greater, and for example, denotes an integer from 1 to 30, from 1 to 20, from 1 to 15, from 1 to 10, from 1 to 8, or from 1 to 6.

Examples of the divalent linking group denoted by Y¹ include -NH-, -O-, -COO-, -O-CO-, -CO-NH-, -NH-CO-, -NH-CO-O-, -O-CO-NH-, -O-CO-O-, and -NH-CO-NH-. R is a group derived from a fluorescent substance or pigment. Here, examples of the fluorescent substance or pigment include quantum dots, Alexa Fluor-350, Alexa Fluor-430, Alexa Fluor-488, Alexa Fluor-532, Alexa Fluor-546, Alexa Fluor-555; Alexa Fluor-568, Alexa Fluor-594, Alexa Fluor-633, Alexa Fluor-647, Alexa Fluor-660, Alexa Fluor-680, Alexa Fluor-750, Cy2, Cy3, Cy3.5, Cy5, Cy5.5, Cy7, BODIPY CR-6G, BOPIPY 530/550, BODIPY FL, BODIPY 505/515, fluorescein isothiocyanate (FITC), eosin isothiocyanate, PE, Rhodamine B, BODIPY 580/605, Texas Red, APC, indocyanine green, 4-acetamido-4'-isothiocyanotostilbene-2,2'-disulfonic acid, acridine, acridine isothiocyanate, 5-(2'-aminoethyl) amino naphthalene-1-sulfonic acid (EDANS), 4-amino-N-[3-vinylsulfonyl) phenyl] naphthalimide-3,5 disulfonate (Lucifer Yellow VS), N- (4-anilino-1-naphthyl) maleimide, anthranilamide, brilliant yellow, coumarin, 7-amino-4-methyl coumarin (AMC), 7-amino-4-trifluoromethyl coumarin (coumarin 151), cyanosine, 4',6-diaminidino-2-phenylindole (DAPI), 5',5"-dibromopyrogallol-sulfonephthalein (Bromopyrogallol Red), 7-diethylamino-3-(4'-isocyanatophenyl)-4-methyl coumarin, diethylene triamine tetraacetic acid, 4,4'-diisothiocyanatodihydro-stilbene-2,2'-disulfonic acid, 4,4'-diisothiocyanatostilbene-2,2'-disulfonic acid, 5- [dimethylamino] naphthalene-1-sulfonyl chloride (DNS), 4-(4'-dimethylaminophenylazo) benzoic acid (DABCYL), 4-dimethylaminophenylazophenyl-4'-isothiocyanate (DABITC), eosin, eosin isothiocyanate, erythrosin, erythrosin B, erythrosin isothiocyanate, ethidium, 5-carboxyfluorescein (FAM), 5-(4,6-dichlorotriazin-2-yl) aminofluorescein (DTAF), 2',7'-dimethoxy-4',5'-dichloro-6-carboxyfluorescein (JOE), fluorescein, hexachloro-6-carboxyfluorescein (HEX), tetrachlorofluorescein (TET), fluorescamine, IR144, IR1446, malachite green isothiocyanate, 4-methylumbelliferone, ortho-cresolphthalein, nitrotyrosine, pararosaniline, phenol red, B-phycoerythrin, R-phycoerythrin, o-phthaldialdehyde, Oregon Green, propidium iodide, pyrene, pyrene butyrate, succinimidyl 1-pyrenebutyrate, Reactive Red 4, 6-carboxy-X-rhodamine (ROX), 6-carboxyrhodamine (R6G), lissamine rhodamine B sulfonyl chloride, rhodamine B, rhodamine 123, rhodamine green, rhodamine X isothiocyanate, sulforhodamine B, sulforhodamine 101, N,N,N',N'-tetramethyl-6-carboxyrhodamine (TAMRA), tetramethylrhodamine, tetramethylrhodamine isothiocyanate (TRITC), riboflavin, rosolic acid, acridine, aniline black, indanthrone, Congo red, methylene blue, neutral red, phenolphthalein, fuchsin, para red, mauve, carotene, xanthophyll, cryptoxanthin, zeaxanthin, fucoxanthin, lycopene, lutein, flavanone, anthochlor, anthocyanin, catechins, melanin, chlorophyll, cytochrome, hemoglobin, hemovanadin, hemocyanin, porphyrins, porphines, myoglobin, phycocyanin, biliverdin, bilirubin, alizarin, anthraquinone, indigo, curcumin, genistein, cochineal, shikonin, tannin, berberine, litmus, and rhodopsin. In the present specification, Alexa Fluor may be abbreviated as "Alexa".

The "group derived from a fluorescent substance or pigment" and denoted by R includes all moieties other than Y¹ derived from the raw materials used when binding the fluorescent substance or pigment. For example:
(i) a carbon nanomaterial having a quantity of n1 NH₂ groups, namely CNM(NH₂)ₙ₁, and
(ii) an N-hydroxysuccinimide ester of an ICG derivative (ICG-NHS) are reacted, and
(iii) a photo-exothermic composite material CNM-(Y¹-R)ₙ₁ is produced according to the following reaction scheme.

[Chem. 1] CNM-(NH₂)ₙ₁ + n1(ICG-NHS) → CXM-(Y¹-R)ₙ₁

(R denotes a group derived from the ICG, n1 is an integer of 1 or greater, and Y¹ is as described below.)

In the case of this reaction, the structures of ICG-NHS, ICG, R, and Y¹ are as illustrated below.

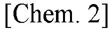

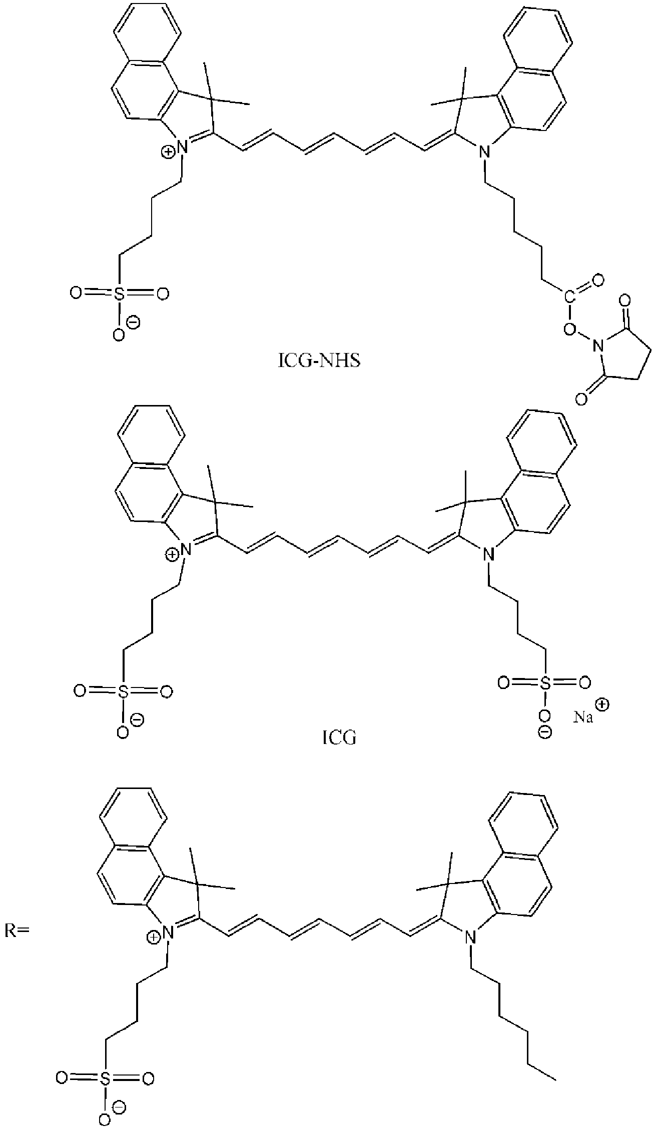

Y¹= NHCO

The above "R" is not the ICG itself, but includes a structure that emits the fluorescence of ICG, and is a group derived from ICG-NHS, which is a reagent for introducing ICG. Thus, "R" corresponds to the "group derived from a fluorescent substance or pigment". The same applies to reagents for introducing fluorescent substances or pigments other than ICG-NHS.

Note that, Y¹(NHCO) is a compound in which an NH derived from an NH₂ group on the CNM surface is bonded with a CO derived from CO-O- (succinimide), which is a reactive group of the ICG-NHS, and Y¹ can be formed by bonding NH, CO, COO, O, or the like derived from a surface group (NH₂, COOH, OH, or the like) of the CNM with an NH, CO, COO, O, CONH or the like derived from a reactive group (NH₂, COOH, OH, NCO, acid halide, acid anhydride, active ester, halogen atom, or the like) of the reagent for introducing the fluorescent substance or pigment.

Also, when the fluorescent substance or pigment itself has a reactive group (NH₂, COOH, OH, or the like) that does not substantially affect the light absorption or fluorescence properties, a photo-exothermic composite material can be obtained by using, as necessary, a condensation agent (dicyclohexylcarbodiimide (DCC), water soluble DCC, carbonyldiimidazole, or the like) to react the CNM with the fluorescent substance or pigment. In this case, the R (group derived from a fluorescent substance or pigment) is a moiety excluding the reactive group participating in the reaction from the fluorescent substance or pigment. Examples of such a case are presented below.

CNM(NH₂)ₙ₁ + R-COOH → CNM(NHCOR)ₙ₁

CNM(NH₂)ₙ₁ + R-NCO → CNM(NHCONHR)ₙ₁

CNM(COOH)ₙ₁ + R-NH₂ → CNM(CONHR)ₙ₁

CNM(COOH)ₙ₁ + R-OH → CNM(CO-OR)ₙ₁

CNM(OH)ₙ₁ + R-COX → CNM(O-COR)ₙ₁

CNM(OH)ₙ₁ + R-NCO → CNM(O-CONHR)ₙ₁

In the schemes above, X denotes OH, a halogen atom (Cl, Br, I), or a group derived from an ester such as O-succinimidyl, maleimide, O-Me, OEt, and O-Bu, R is a moiety excluding a reactive group (NH₂, COOH, OH, COX) from the fluorescent substance or pigment, and corresponds to the group derived from a fluorescent substance or pigment, and n1 is an integer of 1 or greater.

Carbon nanomaterials (CNM) having a large number of groups such as OH, COOH, and NH₂ on the surface are known, and a photo-exothermic composite material according to an embodiment of the present invention can be obtained in accordance with Schemes (1) to (10) below using such carbon nanomaterials.

### Schemes

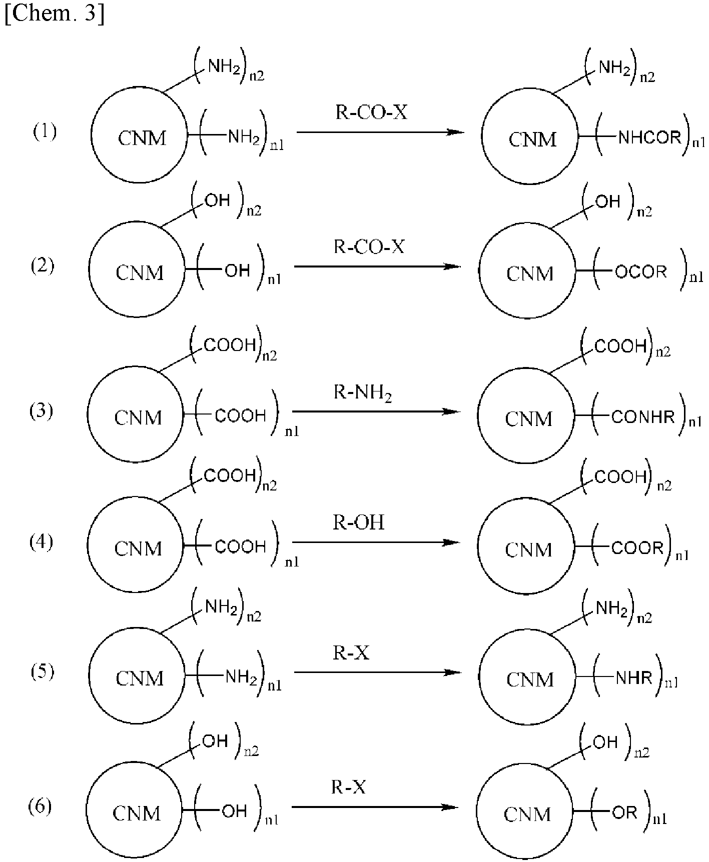

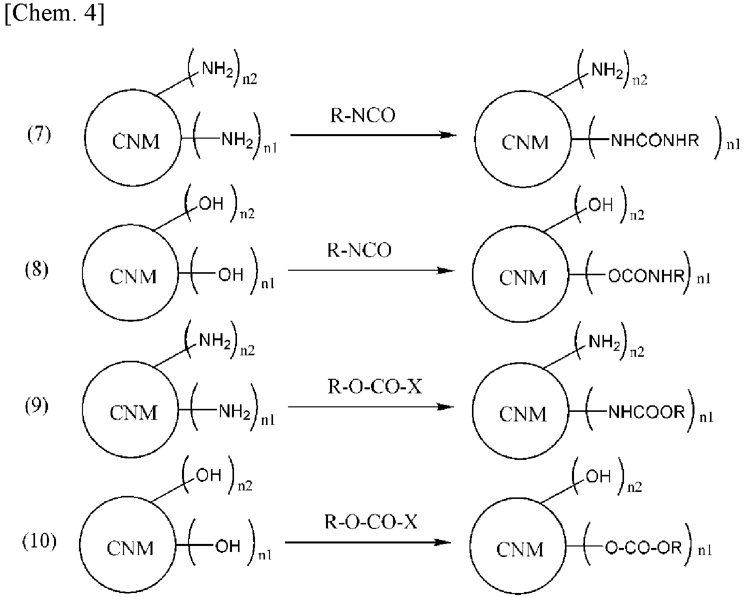

(Wherein, X denotes OH, a halogen atom (Cl, Br, I), or a group derived from an ester such as O-succinimidyl, maleimide, O-Me, OEt, or O-Bu; CNM denotes a carbon nanomaterial; n1 is an integer of 1 or greater; n2 is an integer of 0 or greater; and R is a group derived from a fluorescent substance or pigment.)

The reactions of Schemes (1) to (10) above can be carried out according to a known method, and a targeted product can be obtained by using from 1 mg to an excess amount of any compound selected from R-COX, R-NH₂, R-OH, R-X, R-NCO, and R-O-CO-X per 1 g of a carbon nanomaterial having a surface group of OH, NH₂, or COOH, and carrying out a reaction for 1 to 24 hours at a temperature of from 0°C to the boiling temperature of the solvent. Examples of the solvent include halogenated hydrocarbons such as chloroform, methylene chloride, and 1,2-dichloroethane; aromatic hydrocarbons such as toluene; tetrahydrofuran, diethyl ether, and diisopropyl ether.

The nanoclusters according to an embodiment of the present invention can be produced by suspending a photo-exothermic composite material in a suitable aqueous medium such as water or a buffer solution, and self-assembling through sonication. After sonication, the self-assembled nanoclusters can be separated by removing, through a purification operation such as centrifugation, any photo-exothermic composite material that did not form nanoclusters.

The nanoclusters according to an embodiment of the present invention can be complexed with an active ingredient by being suspended in a suitable solvent containing the active ingredient. The active ingredient is present within or on the surface of the nanoclusters. Examples of the active ingredient include physiologically active substances as well as the fluorescent substances, and pigments.

A nanocluster according to an embodiment of the present invention complexed with an active ingredient gradually releases the active ingredient when administered within or applied to the living body or skin of a mammal (such as a human, mouse, rat, hamster, horse, cow, pig, goat, sheep, rabbit, dog, or cat). When the active ingredient is a physiologically active substance, the nanoclusters function as delivery carriers for the physiologically active substance (drugs in particular). When the active ingredient is a fluorescent substance or pigment, the active ingredient can enhance photo-exothermic properties, and is useful for thermal therapy and photodynamic therapy of cancer. Such an active ingredient is also useful for bioimaging.

Thus, nanoclusters according to an embodiment of the present invention complexed with an active ingredient are useful as pharmaceutical products or pharmaceutical compositions. Examples of dosage forms of pharmaceutical products include tablets, capsules, lozenges, pills, chewable tablets, injectable agents, suppositories, syrups, ointments, and plaster agents. The nanoclusters according to an embodiment of the present invention gradually release the active ingredient, and thus are also useful as delivery carriers of active ingredients.

Physiologically active substances include drugs, nucleic acids, and proteins. Examples of drugs include, but are not limited to, anti-tumor agents, anti-hypertension agents, anti-hypotensive agents, anti-psychotic agents, analgesics, antidepressants, antimanic agents, anti-anxiety agents, sedatives, hypnotics, anti-epileptic agents, opioid agonists, asthma therapeutics, anesthetics, anti-arrhythmia agents, arthritis therapeutic agents, anti-spasmodic agents, ACE inhibitors, decongestants, antibiotics, anti-angina agents, diuretics, anti-Parkinson's disease agents, bronchodilators, antidiuretics, diuretics, anti-hyperlipidemia agents, immunosuppressive agents, immunomodulatory agents, anti-emetics, anti-infective agents, anti-neoplastic agents, antifungal agents, antiviral agents, anti-diabetic agents, anti-allergenic agents, antipyretics, gout suppressants, antihistamines, anti-pruritic agents, bone regulators, cardiovascular agents, cholesterol lowering agents, antimalarials, antitussives, expectorants, mucolytic agents, nasal packing agents, dopamine agonists, gastrointestinal agents, muscle relaxants, neuromuscular blockers, parasympathomimetic agents, prostaglandins, stimulants, appetite suppressors, thyroid or anti-thyroid agents, hormones, anti-migraine agents, anti-obesity agents, and antiinflammatory agents. A preferred drug is an anti-tumor agent. Examples of anti-tumor agents include hormonal therapeutic agents (e.g., fosfestrol, diethylstilbestrol, chlorotrianiserin, medroxyprogesterone acetate, megestrol acetate, chlormadinone acetate, cyproterone acetate, danazol, allylestrenol, gestrinone, mepartricin, raloxifene, ormeloxifene, levormeloxifene, anti-estrogens (e.g., tamoxifen citrate, toremifene citrate), pill preparations, mepitiostane, testolactone, aminoglutethimide, LH-RH agonists (e.g., goserelin acetate, buserelin, leuprorelin), droloxifene, epitiostanol, ethynyl estradiol sulfonate, aromatase inhibitors (e.g., fadrozole hydrochloride, anastrozole, letrozole, exemestane, borazole, formestane), antiandrogens (e.g., flutamide, bicalutamide, nilutamide), 5α-reductase inhibitors (e.g., finasteride, epristeride), corticosteroids (e.g., dexamethasone, prednisolone, betamethasone, triamcinolone), androgen synthesis inhibitors (e.g., abiraterone), and retinoids and agents that slow the metabolism of retinoids (e.g., liarozole), and among these, LH-RH agonists (e.g., goserelin acetate, buserelin, leuprorelin)), alkylating agents (e.g., nitrogen mustards, nitrogen mustard-N-oxide hydrochloride, chlorambutyl, cyclophosphamide, ifosfamide, thiotepa, carboquone, improsulfan tosilate, busulfan, nimustine hydrochloride, mitobronitol, melphalan, dacarbazine, ranimustine, estramustine sodium phosphate, triethylene melamine, carmustine, lomustine, streptozocin, pipobroman, etoglucid, carboplatin, cisplatin, miboplatin, nedaplatin, oxaliplatin, altretamine, ambamustine, dibrospidium hydrochloride, fotemustine, prednimustine, pumitepa, ribomustin, temozolomide, treosulfan, trofosfamide, zinostatin stimalamer, carboquone, adozelesin, cystemustine, bizelesin), anti-metabolites (e.g., mercaptopurine, 6-mercaptopurine riboside, thioinosine, methotrexate, enocitabine, cytarabine, cytarabine ocfosfate, ancitabine hydrochloride, 5-FU-based drugs (e.g., fluorouracil, tegafur, UFT, doxifluridine, carmofur, gallocitabine, and emitefur), aminopterin, leucovorin calcium, tabloid, butocine, calcium folinate, calcium levofolinate, cladribine, emitefur, fludarabine, gemcitabine, hydroxycarbamide, pentostatin, piritrexim, idoxuridine, mitoguazone, tiazofurin, and ambamustine), anti-cancer antibiotics (e.g., actinomycin D, actinomycin C, mitomycin C, chromomycin A3, bleomycin hydrochloride, bleomycin sulfate, pepromycin sulfate, daunorubicin hydrochloride, doxorubicin hydrochloride, aclarubicin hydrochloride, pirarubicin hydrochloride, epirubicin hydrochloride, neocarzinostatin, misramycin, sarkomycin, carzinophilin, mitotane, xorubicin hydrochloride, mitoxantrone hydrochloride, and idarubicin hydrochloride), plant-derived anti-cancer agents (e.g., etoposide, etoposide phosphate, vinblastine sulfate, vincristine sulfate, vindesine sulfate, teniposide, paclitaxel, docetaxel, vinorelbine, camptothecin, irinotecan hydrochloride), biological response modifiers (BRM) (e.g., picibanil, Krestin, schizophyllan, lentinan, ubenimex, interferon, interleukin, macrophage colony stimulating factor, granulocyte colony stimulating factor, erythropoietin, lymphotoxin, BCG vaccine, corynebacterium parvum, levamisole, polysaccharide-K, and procodazol), and drugs that inhibit cell growth factors and the action of receptors thereof (e.g., antibody drugs such as trastuzumab (Herceptin (trade name); anti-HER2 antibody), ZD1839 (Iressa), and Gleevec). Examples of the cancers targeted by anti-tumor agents include colorectal cancer, liver cancer, kidney cancer, head and neck cancer, esophageal cancer, stomach cancer, biliary cancer, gallbladder/bile duct cancer, pancreatic cancer, lung cancer, breast cancer, ovarian cancer, cervical cancer, uterine cancer, bladder cancer, prostate cancer, testicular tumors, bone and soft tissue sarcomas, leukemia, malignant lymphoma, multiple myeloma, skin cancer, and brain tumors. Preferable examples includes colorectal cancer, stomach cancer, head and neck cancer, lung cancer, breast cancer, pancreatic cancer, biliary cancer, and liver cancer.

The nucleic acids are not particularly limited and may be DNA, RNA, chimeric nucleic acids of DNA and RNA, or hybrids of DNA/RNA. Further, the nucleic acid can be any of 1 to 3 strands, but is preferably single-stranded or double-stranded. The nucleic acid may be another nucleotide that is an N-glycoside of a purine or pyrimidine base, or another oligomer having a non-nucleotide skeleton (e.g., a commercially available peptide nucleic acid (PNA), etc.) or other oligomer with a special bond (provided that the oligomer contains a nucleotide having an arrangement that allows pairing of bases or attachment of bases as found in DNA or RNA). The nucleic acid may also be one with a known modification. Examples include labeled nucleic acids known in the relevant technical field, capped nucleic acids, methylated nucleic acids, nucleic acids having one or more natural nucleotides substituted with an analog, nucleic acids subjected to intramolecular nucleotide modifications, for example, those with uncharged bonds (e.g., methylphosphonates, phosphotriesters, phosphoramidates, and carbamates, etc.), nucleic acids having a charged bond or a sulfur-containing bond (e.g., phosphorothioate, and phosphorodithioate, etc.), for example, nucleic acids having a side chain group such as a protein (nuclease, nuclease inhibitor, toxin, antibody, and signal peptide, etc.) or sugars (e.g., monosaccharides, etc.), nucleic acids having an intercalated compound (e.g., acridine and psoralen, etc.), nucleic acids having a chelate compound (e.g., metals, radioactive metals, boron, oxidative metals, etc.), nucleic acids containing an alkylating agent, and nucleic acids with modified bonds (e.g., α-anomeric nucleic acids, etc.). Preferred nucleic acids include RNA such as siRNA.

An siRNA is a double-stranded oligo RNA consisting of a nucleotide sequence of the mRNA or initial transcript of a target gene or a nucleotide sequence homologous to a partial sequence (preferably within the coding region) of the nucleotide sequence thereof (including the intron portion in the case of an initial transcript), and the complementary strand thereof. The length of the portion that is homologous to the target nucleotide and is contained in the siRNA is usually greater than or equal to approximately 18 bases, and for example, the length may be approximately 20 bases (typically, an approximate length from 21 to 23 bases). However, the length thereof is not particularly limited as long as RNA interference can be caused. Ordinarily, the total length of the siRNA is approximately greater than or equal to 18 bases, and for example, the total length may be approximately 20 bases (typically, an approximate total length from 21 to 23 bases). However, the total length is not particularly limited as long as RNA interference can be caused.

The relationship between the target nucleotide sequence and the sequence homologous thereto and contained in the siRNA may be a 100% match, or base mutations may be present (within a range of sameness of at least 70%, preferably 80% or higher, more preferably 90% or higher, and most preferably 95% or higher).

The siRNA may have, at the 5' or 3' end, additional bases that consist of no more than 5 bases, and preferably 2 bases, and which do not form a base pair. The additional base may be DNA or RNA, but when DNA is used, the stability of the siRNA can be improved. Examples of sequences of such additional bases include, but are not limited to, ug-3', uu-3', tg-3', tt-3', ggg-3', guuu-3', gttt-3', ttttt-3', and uuuuu-3'.

The siRNA may be for any target gene. The siRNA is preferably one that targets genes for which an enhanced expression is involved in the onset and/or exacerbation of a disease of interest, and more specifically, is one that targets genes for which an antisense nucleic acid to the gene thereof has advanced to a clinical or preclinical stage, or newly known genes.

The siRNA may be used singly, or in a combination of two or more .

Examples of proteins include enzymes, receptors, antibodies, antigens, and cytokines such as interferons and interleukins.

The approximate average particle diameter of the nanocluster according to an embodiment of the present invention is from 1 to 1000 nm, preferably from 3 to 800 nm, more preferably from 5 to 500 nm, even more preferably from 10 to 300 nm, and particularly from 30 to 250 nm.

The approximate zeta potential of the nanocluster is preferably from 5 to 30 mV, and more preferably from 10 to 25 mV.

When the nanoclusters according to one preferable embodiment of the present invention are complexed with an active ingredient, the approximate content of the active ingredient is preferably from 5 to 50 parts by mass, and more preferably from 10 to 20 parts by mass, per 100 parts by mass of the nanoclusters.

### Examples

The present invention will be described in detail below based on examples, but the present invention is not limited to these examples.

### Example 1

### <Experimental Method>

(1) Synthesis of ND Super Particle Complex: ND (diameter: from 4 to 5 nm) were synthesized according to a previously reported preparation method (V V. Danilenko, Combust., Explos. Shock Waves 2005, 41, 577; V. Y. Dolmatov, J. Superhard Mater. 2008, 30, 233; V. Y, Dolmatov, V. Myllymaki and A. Vehanen, J. Superhard Mater. 2013, 35, 143.), The synthesized ND were purified by nitric acid and fired in a hydrogen gas atmosphere. The elements of the ND [contents of C (92.20%), H (0.74%), and N (2.30%)] were analyzed using an organic element analyzer (Micro Corder JM10; J-Science Lab Co., Ltd, Kyoto, Japan). The purified ND were dispersed in distilled water by a bead mill (Sand Grinder LSG-4U; Aimex Co., Ltd, Tokyo, Japan). Next, the ND dispersion was centrifuged to remove the ND insoluble in water. The resulting supernatant dispersion (ND-ori) was used in an additional experiment. 0.1 ml of the ND-ori dispersion (ND concentration = 30 mg ml⁻¹) and 1 mg of ICG-NHS (Goryo Chemical, Hokkaido, Japan) were dissolved in 5 ml of a 2-(N-morpholino)ethane sulfonic acid (MES) buffer solution (pH 6.0, 100 mM) by sonicating for 5 minutes using a bus-type sonicator (output: 80 W, oscillation frequency: 40 kHz) (USD-2R; AS ONE, Osaka, Japan). The mixture was additionally stirred vigorously with a stirrer for 1.5 hours at room temperature, and then washed three times with Mill-Q water using centrifugation, and an unreacted material was thus removed. Pellets obtained by centrifugation were re-dispersed in 1 ml of Mill-Q water by irradiating with ultrasonic waves for 10 minutes using a pulsed sonicator (VCX-600; Sonics, Danbury, CT, USA). The ICG-ND-SP dispersion was used in a subsequent experiment. In addition, the ICG concentration (up to 0.75 mg ml⁻¹) and the ND concentration (up to 3 mg ml⁻¹) in the ICG-ND-SP dispersion were estimated using an UV-Vis-NIR spectrophotometer (V-730 BIO; Jasco, Tokyo, Japan) and thermogravimetric (TGA) measurements (using the Q500 thermogravimetric analyzer; TA Instruments, New Castle, DE, USA).

BODIPY-ND-SP and Alexa568-ND-SP were synthesized by the same method used to synthesize the ICG-ND-SP. Specifically, BODIPY FL NHS Ester (Succinimidyl Ester) (1 mg; Invitrogen, Carlsbad, CA, USA) or Alexa Fluor 568 NHS Ester (Succinimidyl Ester) (1 mg; Invitrogen) was added to the reaction solution instead of ICG-NHS. After preparation, it was confirmed through measurements with a UV-Vis-NIR spectrophotometer that BODIPY (ca. 0.04 mg ml⁻¹) and Alexa Fluor 568 molecules (ca. 0.04 mg ml⁻¹) covalently bonded to the ND surface (ca. 3 mg ml⁻¹).

PBA-ND-SP was basically synthesized by the same method as the other fluorescent molecule-modified ND-SP with the exception that the weight of the fluorescent molecules used for synthesis, the addition of water soluble carbodiimide (WSC), the volume of the reaction solution, and the reaction time were different. Namely, 1 ml of a ND-ori solution (ND concentration = 30 mg ml⁻¹), 10 mg of 4-phenylbutyric acid (PBA) (Tokyo Chemical Industry, Tokyo, Japan), and 10 mg of WSC were added to 9 ml of an MES buffer solution (pH 6.0, 100 mM) and irradiated with ultrasonic waves for 20 minutes using a bus-type sonicator until the PBA was fully dissolved. The reaction solution was then vigorously stirred using a stirrer at room temperature for 1.5 hours, after which the solution was centrifuged and washed three times with Milli-Q water, and unreacted material was thus removed. The obtained pellets were re-dispersed in 10 ml of Mill-Q water through sonication for 10 minutes using a pulsed sonicator (VCX-600: Sonics, Danbury, CT, USA). The obtained PBA-ND-SP dispersion (PBA concentration: up to 0.15 mg ml⁻¹, ND concentration: up to 3 mg ml⁻¹) was used for further experiments.

APTX@ICG-ND-SP complex was prepared by the following method. 10 mg of PTX (Wako) and 10 ml of Milli-Q were added to the washed ICG-ND-SP pellets, after which the mixture was sonicated for 10 minutes using a pulsed sonicator. The resulting mixture was washed with Milli-Q water using a centrifuge and then stored at 4°C until used. PTX@ND-ori was prepared by sonicating 10 mg of PTX, 1 ml of ND-ori, and 9 ml of Milli-Q water using a pulsed sonicator for 10 minutes. Abraxane was purchased from Taiho Pharmaceutical Co., Ltd. and used as is in experiments without any treatment such as chemical modification.

(2) Characterization of ND-SP: The structure and morphology of the prepared ND-SP were measured using a high resolution transmission electron microscope (TEM) (EM-002B; Topcon, Tokyo, Japan) at an accelerating voltage of 120 kV

The hydrodynamic diameter of the ND-SP was measured by dynamic light scattering (DLS) (using the Photal FPAR-1000; Otsuka Electronics, Osaka, Japan).

The spectral profile and concentration of a complex of ND-SP, fluorescent pigment, and PTX were measured using a UV-Vis-NIR spectrophotometer.

The fluorescence properties of the ND-SP, fluorescent pigment, and ND-ori were analyzed using a fluorometer (FP-6500 or FP-8500; Jasco, Tokyo) and a microplate reader (Infinite M200 PRO; Tecan, Mannedorf, Switzerland).

(3) Cell Culture and Cytotoxicity Evaluation: Human osteosarcoma cells (U2OS) and human ovarian adenoma cells (SKOV3) were obtained from the Japanese Collection of Research Bioresources Cell Bank (Tokyo, Japan), and were cultured with Dulbecco's Modified Eagle's Medium (DMEM) (Gibco, Grand Island, NY, USA) containing 10% fetal bovine serum, 2 mM L-glutamine, 1 mM sodium pyruvate, gentamycin, penicillin-streptomycin (100 IU ml⁻¹), and Hank's balanced salt solution (Life Technologies, Carlsbad, CA, USA). The cells were cultured in a humidified chamber at 37°C with a 5% CO₂ atmosphere.

Cell viability was evaluated using the Cell Counting Kit (CCK)-8 (Dojindo Laboratories, Kumamoto, Japan) according to the manual thereof. The cells were seeded onto a 96-well plate (× 10³ cells well⁻¹) and incubated overnight. Next, the cells were exposed to a drug or nanocomplex dispersion solution and washed with a fresh culture solution, and then incubated in a CCK-8 solution. Finally, cell viability was calculated by measuring the absorbance at 450 nm with a microplate reader.

(4) Fluorescence Microscope Imaging: U2OS cells were seeded onto a glass bottom dish for imaging and incubated overnight. An ND-SP dispersed aqueous solution with various chemically-modified fluorescent pigments was added to cells adhered to the bottom of the dish, and the cells were incubated for 6 hours or 24 hours at 37°C in a 5% CO₂atmosphere. The nucleus of each cell was stained for 10 minutes using Hoechst 33342 (1 µg ml⁻¹; Thermo Fisher Scientific, Waltham, MA, USA). After three washes using a PBS buffer solution, the cells were maintained in an RPMI 1640 Phenol Red-free medium (Thermo Fisher Scientific) for live imaging. When imaging using PBA-ND-SP, the actin (cytoskeleton) was stained using Phallodin (Thermo Fisher Scientific). In immobilized imaging, the cells were stained for 10 minutes at room temperature using a 4% paraformaldehyde solution or a pre-chilled methanol/acetone mixture (v:v of 1:1), after which the cells were washed with a PBS buffer solution and then observed. Near infrared (NIR) fluorescence images of the ICG-ND-SP were observed using a fluorescence microscope equipped with a mirror unit (IRDYE800-33LP-A-U01; Semrock) and an electronically-magnifying CCD camera (DP80; Olympus). A confocal laser microscope (LSM 5 PASCAL, Carl Zeiss Inc., Tokyo, Japan) was used for intra-cellular imaging of the PBA-ND-SP, BODIPY-ND-SP, and Alexa568-ND-SP complexes.

(5) Temperature Measurements: The temperature change of the laser irradiated ICG-ND-SP dispersion was examined in the following manner. Milli-Q water (200 µl, ICG and ND concentrations of 0.75 mg ml⁻¹ and 3 mg ml⁻¹, respectively) in which ICG-ND-SP was dispersed was added to a quartz cell (GL Science, Tokyo, Japan). The sample was irradiated for 5 minutes with a fiber-coupled continuous wave laser (laser spot diameter: up to 4 mm; maximum power: 1 W, up to 80 mW mm⁻²; BRM-785-1.0-100-0.22-SMA; B&W Tek, Newark, DE, USA) at a wavelength of 785 nm. Similarly, a PBS buffer solution not containing ICG-ND-SP was also laser irradiated as a control. The temperature change of the solution upon laser irradiation was measured using a temperature sensor (AD-5601A; A&D, Tokyo, Japan). The ICG and ND concentrations in the ICG-NHS and ND-ori solutions were adjusted in advance to be the same as the ICG and ND concentrations in the ICG-ND-SP complex measured using a UV-Vis-NIR spectrophotometer.

The effect of the concentrations of the ICG and ND on the photo-exothermic behavior of the ICG-ND-SP complex was examined by diluting the ICG-ND-SP dispersion (stock solution) with a DMEM culture medium.

(6) ROS Detection: ROS analysis was implemented using a 96-well plate (Thermo Fisher Scientific) with a transparent bottom and a black main body, and Singlet Oxygen Sensor Green (SOSG) (Invitrogen) as a singlet oxygen detection reagent. Milli-Q water (100 µL) in which ICG-ND-SP was dispersed was diluted with a PBS buffer solution containing the SOSG. The final concentrations in the system of ICG, ND, and SOSG were 75 µg ml⁻¹, 300 µg ml⁻¹, and 1 µM, respectively. Next, the sample was irradiated for 5 minutes with a near-infrared laser having a wavelength of 785 nm at an output of 1 W (ca. 80 mW mm⁻²). A PBS buffer solution not containing a nanocomplex was used as a control. Green fluorescence associated with ROS generation was measured using a microplate reader (excitation wavelength: 485 nm, fluorescence wavelength: 535 nm).

(7) Cancer Cell Elimination Effect through Laser-Irradiated ICG-ND-SP: SKOV3 cells (5 × 10³ cells/well) were seeded onto a 96-well plate and incubated overnight to evaluate cell viability. The cells were treated with 100 µl of a DMEM culture medium containing various samples (ND-ori, ICG, ICG-ND-SP, PTX@ICG-ND-SP) or a PBS buffer solution not containing a nanocomplex, and then irradiated with a laser (wavelength of 785 nm, output of 1 W, up to 80 mW mm⁻²). After laser irradiation, the cells were washed and incubated in a fresh culture medium. The cell viability of samples immediately after laser irradiation and of samples incubated for 24 hours after laser irradiation was examined using a CCK-8 kit. Note that the concentrations of ICG and ND in the system were carefully adjusted to 25 and 100 µg ml⁻¹.

(8) In vivo Anticancer Assays: Female BALB/cAJc-nu/nu mice (n = 4; 8 weeks old, average body weight = 18 g) were obtained from Japan SLC (Hamamatsu, Japan) and were raised in a Specific Pathogen Free (SPF) environment in which specific pathogens were removed. Animal experiments were conducted according to an animal experiment plan that was reviewed by the Animal Experiment Ethics Committee of the National Institute of Advanced Industrial Science and Technology (AIST) and approved by the chairman of AIST. Cancer model mice bearing SKOV3 cells were created by injecting, into both thighs of the mice, 100 µl of a mixed solution (v/v of 1:1) of culture medium/Matrigel (Dow Corning, Corning, NY, USA) containing a quantity of 1 × 10⁶ cells. After 2 weeks, 200 µl of various samples [sterile water containing ICG-ND-SP (ICG, 7.5 mg kg⁻¹; ND-ori, 30 mg kg⁻¹), 10% Cremophor EL (Sigma-Aldrich) containing PTX (5 mg kg⁻¹), sterile water containing PTX@ICG-ND-SP (PTX, 5 mg kg⁻¹; ICG, 7.5 mg kg⁻¹; ND-ori, 30 mg kg⁻¹), and a PBS buffer solution (control)] were administered once every two days into the abdominal cavity of the mice for which the cancer volume had reached 50 mm³. The size of the cancer and the health status (mouse body weight) were monitored once every two days.

(9) In vivo Multiple Cancer Therapy: A mixed solution (v/v of 1:1) of culture medium/Matrigel (Dow Corning, Corning, NY, USA) containing SKOV3 cells (1 × 10⁶) was subcutaneously administered into both sides of the backs of 8-week old female nude mice (average body weight = 18 g, N = 3; BALB/cSlc-nu/nu, Japan SLC). Three weeks after implantation, the mice were divided into four groups of three mice each, and 50 µl of various samples [sterile water containing PBS and ICG-ND-SP (ICG, 1.88 mg kg⁻¹; ND-ori, 7.5 mg kg⁻¹), sterile water containing ND-ori (7.5 mg kg⁻¹), and sterile water containing PTX@ICG-ND-SP (PTX, 1.25 mg kg⁻¹; ICG, 1.88 mg kg⁻¹; ND-ori, 7.5 mg kg⁻¹) was administered by syringe into the solid cancers. Only the solid cancer on the right side was irradiated for 3 minutes once every three days using a near infrared laser [wavelength 785 nm, output (1 W, up to 80 mW/mm²)]. Note that when the size of the solid cancer was greater than the laser spot (diameter of up to 4 mm), two locations on the surface of the solid cancer were irradiated by laser for 3 minutes each. The body surface of each mouse when irradiating by laser was measured using IR thermography (i7; FLIR, Nashua, NH, USA). In the same manner as with the in vivo anticancer assays, the size of the cancer and the health status (mouse body weight) were monitored once every two days.

(10) In Vivo Distribution of ND-SP in Cancer Model Mice: 200 µl of sterile water containing PTX@ICG-ND-SP (PTX, 5 mg kg⁻¹; ICG, 7.5 mg kg⁻¹; ND-ori, 30 mg kg⁻¹) was injected into the caudal vein of cancer model mice implanted with SKOV3. Near infrared fluorescence images of the mice and tumor tissues were measured using the IVIS Imaging Spectrum System (PerkinElmer, MA, USA). The excitation wavelength and the fluorescence wavelength were λₑₓ = 740 nm and λₑₘ = 800 nm, respectively. The images were analyzed using IVIS Living Imaging 3.0 software (PerkinElmer).

(11) Blood Tests: A complete blood count (CBC) and biochemical parameters were measured by Japan SLC and Oriental Yeast Co. (Tokyo, Japan). Specifically, 200 µl of various samples [sterilized water containing ICG-ND-SP (ICG, 7.5 mg kg⁻¹; ND-ori, 30 mg kg⁻¹) or a PBS buffer solution] were administered through the caudal vein to 10-week old female BALB/cSlc mice (n = 5, average body weight = 21 g; Japan SLC). Blood samples were drawn at 7 days and 28 days after administration of the nanocomplex. The results are shown in Tables 1 and 2.

[Table 1]

**Complete Blood Count (CBC) and Biochemical Parameters of Mice One Week After Injection of PBS or ICG-ND-SP**

| Method | Item | Units | PBS (n=5) | IGG-ND-SP (n=5) | p-value |
|---|---|---|---|---|---|
| CBC | WBC | ×10² µl⁻¹ | 39+9 | 42±12 | >0.05 |
| | RBC | ×10⁴ µl⁻¹ | 917+52 | 895+32 | >0.05 |
| | PLT | ×10⁴ µl⁻¹ | 60+3 | 65+2 | >0.05 |
| Biochemical Parameters | CRP | µg ml⁻¹ | 1.1±0.1 | 1.0±0.1 | >0.05 |
| | TP | g dl⁻¹ | 4.0±0.1 | 4.1+0.3 | >0.05 |
| | ALB | g dl⁻¹ | 2.7±0.1 | 2.7+0.3 | >0.05 |
| | BUN | mg dl⁻¹ | 23+2 | 21±1 | >0.05 |
| | CRE | mg dl⁻¹ | 0.11+0.02 | 0.09+0.02 | >0.05 |
| | AST | IU l⁻¹ | 60±5 | 67±17 | >0.05 |
| | LDH | IU l⁻¹ | 325+39 | 349+95 | >0.05 |
| | AMY | IU l⁻¹ | 1837+173 | 1772±136 | >0.05 |
| | CK | IU l⁻¹ | 359±165 | 266±187 | >0.05 |

| | | | | | |
|---|---|---|---|---|---|
| Abbreviations: ALB, albunim; AMY, amylase; AST, aspartate aminotransferase; BUN, blood urea nitrogen; CK, creatine kinase; CRE, creatinine; CRP, C-reactive protein; LDH, lactate dehydrogenase; PLT, platelet; RBC, red blood cell; TP, total protein; WBC, white blood cell. | | | | | |

[Table 2]

**Complete Blood Count (CBC) and Biochemical Parameters of Mice Four Weeks After Injection of PBS or ICG-ND-SP**

| Method | Item | Units | PBS (n=5) | IGG-ND-SP (n=5) | p-value |
|---|---|---|---|---|---|
| CBC | WBC | ×10² µl⁻¹ | 46+8 | 42+8 | >0.05 |
| | RBC | ×10⁴ µl⁻¹ | 897+37 | 906+40 | >0.05 |
| | PLT | ×10⁴ µl⁻¹ | 65+6 | 61+5 | >0.05 |
| Biochemical Parameters | CRP | µg ml⁻¹ | 1.0+0.2 | 1.0+0.2 | >0.05 |
| | TP | g dl⁻¹ | 3.8+0.2 | 3.8+0.2 | >0.05 |
| | ALB | g dl⁻¹ | 2.6±0.1 | 2.6±0.1 | >0.05 |
| | BUN | mg dl⁻¹ | 17+4 | 17+4 | >0.05 |
| | CRE | mg dl⁻¹ | 0.11±0.01 | 0.12+0.02 | >0.05 |
| | AST | IU l⁻¹ | 59+15 | 59±13 | >0.05 |
| | LDH | IU l⁻¹ | 261±118 | 250+77 | >0.05 |
| | AMY | IU l⁻¹ | 1575+74 | 1686±135 | >0.05 |
| | CK | IU l⁻¹ | 228+137 | 216+149 | >0.05 |

| | | | | | |
|---|---|---|---|---|---|
| Abbreviations: ALB, albunim; AMY, amylase; AST, aspartate aminotransferase; BUN, blood urea nitrogen; CK, creatine kinase; CRE, creatinine; CRP, C-reactive protein; LDH, lactate dehydrogenase; PLT, platelet; RBC, red blood cell; TP, total protein; WBC, white blood cell. | | | | | |

### Statistical Analysis of Data

In the data, ± indicates the standard deviation, and n denotes the number of samples that were used. The Student's t-test method was used for statistical analysis of data. The symbols ^{∗}, ^{∗∗}, and ^{∗∗∗} indicate p values of <0.05, <0.005, and <0.001, respectively.

## Claims

1. A photo-exothermic composite material represented by Formula (I):
CNM-(Y¹-R)ₙ₁ (I)
wherein, CNM denotes a carbon nanomaterial; Y¹ denotes a divalent linking group; R denotes a group derived from a fluorescent substance or pigment; and n1 is an integer of 1 or greater.

2. The photo-exothermic composite material according to claim 1, wherein the divalent linking group represented by Y¹ is selected from the group consisting of - NH-, -O-, -CO-O-, -O-CO-, -CO-NH-, -NH-CO-, -NH-CO-O-, -O-CO-NH-,-O-COO-, and -NH-CO-NH-.

3. The photo-exothermic composite material according to claim 1, wherein the carbon nanomaterial is a carbon nanodiamond or carbon nanodots.

4. The photo-exothermic composite material according to claim 3, wherein the carbon nanomaterial is a carbon nanodiamond.

5. The photo-exothermic composite material according to any one of claims 1 to 4, wherein the carbon nanomaterial has at least one surface group selected from the group consisting of OH, COOH, and NH₂, and the group derived from a fluorescent substance or pigment and denoted by R is bonded to the carbon nanomaterial through the surface group.

6. A nanocluster in which the photo-exothermanyic composite material described in any of claims 1 to 5 is self-assembled.

7. The nanocluster according to claim 6, further complexed with an active ingredient.

8. The nanocluster according to claim 7, wherein the active ingredient is a physiologically active substance, a fluorescent substance, or a pigment.

9. A delivery carrier for an active ingredient, the delivery carrier comprising the nanocluster described in claim 7 or 8.

10. A pharmaceutical composition comprising the nanocluster described in claim 7 or 8, the nanocluster being complexed with a drug.
